(19) Europäisches Patentamt
European Patent Office
Office européen des brevets

(11) Veröffentlichungsnummer: **0 537 539 A1**

(12) **EUROPÄISCHE PATENTANMELDUNG**

(21) Anmeldenummer: **92116538.7**

(51) Int. Cl.5: **C07D 263/58**, A01N 43/76

(22) Anmeldetag: **28.09.92**

(30) Priorität: **11.10.91 DE 4133673**

(43) Veröffentlichungstag der Anmeldung: **21.04.93 Patentblatt 93/16**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Förster, Heinz, Dr.**
**Am Eckbusch 47**
**W-5600 Wuppertal 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **2-(2-Benzoxazolyl-oxy)-acetamide und ihre Verwendung als Herbicides.**

(57) Die Erfindung betrifft neue 2-(2-Benzoxazolyl-oxy)-acetamide der Formel (I),

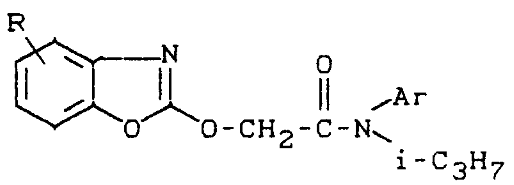

(I)

in welcher
R für Wasserstoff, Halogen, Alkyl oder Halogenalkyl steht und
Ar für gegebenenfalls substituiertes Aryl steht,
wobei jedoch die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind,
ein Verfahren zu ihrer Herstellung und ihre Verwendung als Herbizide.

EP 0 537 539 A1

Die Erfindung betrifft neue 2-(2-Benzoxazolyl-oxy)-acetamide, ein Verfahren zu ihrer Herstellung und ihre Verwendung als Herbizide.

Es ist bekannt, daß bestimmte 2-(2-Benzoxazolyl-oxy)-acetamide, wie beispielsweise die Verbindung 2-(6-Chlorbenzoxazol-2-yl-oxy)-N-isopropyl-acetanilid, herbizide Eigenschaften besitzen (vgl. z.B. DE-A 29 03 966; EP-A 5501; US-A 4 509 971; US-A 4 833 243; DE-A 30 38 599; DE-A 30 38 652; DE-A 34 18 168; EP-A 161 602; US-A 4 784 682; DE-A 37 24 467).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 2-(2-Benzoxazolyl-oxy)-acetamide der allgemeinen Formel (I),

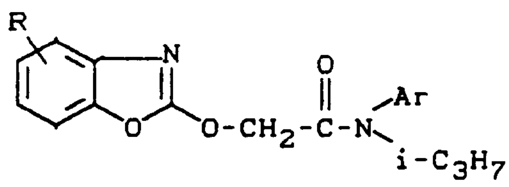

(I)

in welcher

R für Wasserstoff, Halogen, Alkyl oder Halogenalkyl steht und

Ar für gegebenenfalls substituiertes Aryl steht,

wobei jedoch die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind,

gefunden.

Weiterhin wurde gefunden, daß man die neuen 2-(2-Benzoxazolyl-oxy)-acetamideder allgemeinen Formel (I) erhält, wenn man Benzoxazol-Derivate der Formel (II),

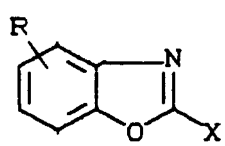

(II)

in welcher

X für Halogen oder Alkylsulfonyl steht und

R die oben angegebene Bedeutung hat,

mit 2-Hydroxyacetamiden der Formel (III)

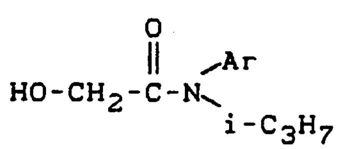

(III)

in welcher

Ar die oben angegebene Bedeutung hat,

gegebenenfalls in Gegenwart eines Verdünnungsmittels und gegebenenfalls in Gegenwart eines Reaktionshilfsmittels umsetzt.

Schließlich wurde gefunden, daß die neuen 2-(2-Benzoxazolyl-oxy)-acetamide der allgemeinen Formel (I) herbizide Eigenschaften besitzen.

Überraschenderweise zeigen die erfindungsgemäßen neuen 2-(2-Benzoxazolyl-oxy)-acetamide der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten 2-(2-Benzoxazolyl-oxy)-acetamiden, wie beispielsweise 2-(6-Chlorbenzoxazol-2-yl-oxy)-N-isopropylacetanilid.

Die erfindungsgemäßen neuen 2-(2-Benzoxazolyl-oxy)-acetamide sind durch die Formel (I) allgemein definiert. Bevorzugt sind Verbindungen der Formel (I), bei welchen

R für Wasserstoff, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

Ar für jeweils gegebenenfalls einfach oder mehrfach, gleich oder verschieden substituiertes Phenyl oder Naphthyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 6 Kohlenstoffatomen und 1 bis 13 gleichen oder verschiedenen Halogenatomen,

wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

Besonders bevorzugt sind Verbindungen der Formel (I), bei welchen

R für Wasserstoff, Fluor, Chlor, Brom, für geradkettiges oder verzweigtes Alkyl mit 1 bis 6 Kohlenstoffatomen oder für geradkettiges oder verzweigtes Halogenalkyl mit 1 bis 3 Kohlenstoffatomen und 1 bis 7 gleichen oder verschiedenen Halogenatomen steht und

Ar für gegebenenfalls einfach bis dreifach, gleich oder verschieden substituiertes Phenyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

Ganz besonders bevorzugt sind Verbindungen der Formel (I), bei welchen

R für Wasserstoff, Chlor, Methyl, Ethyl, n- oder i-Propyl, n-, i-, s- oder t-Butyl oder Trifluormethyl steht und

Ar für gegebenenfalls einfach oder zweifach, gleich oder verschieden substituiertes Phenyl steht, wobei als Substituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy und/oder Trifluormethylthio,
wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

Im einzelnen sei auf die bei den Herstellungsbeispielen genannten Verbindungen verwiesen.

Verwendet man beispielsweise 2-Chlor-5-methylbenzoxazol und 2-Hydroxy-N-isopropyl-acetanilid als Ausgangsstoffe, so läßt sich der Reaktionsablauf des erfindungsgemäßen Verfahrens durch das folgende Formelschema darstellen:

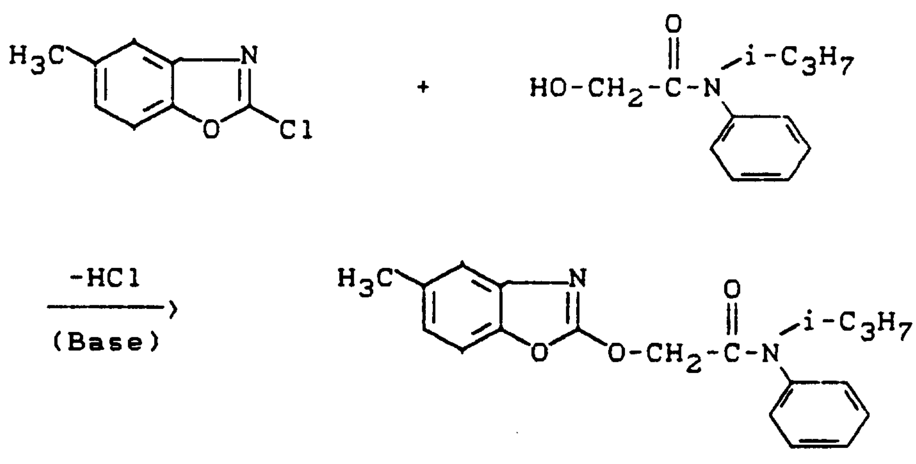


Die zur Durchführung des erfindungsgemäßen Verfahrens als Ausgangsstoffe benötigten Benzoxazol-Derivate sind durch die Formel (II) allgemein definiert. In dieser Formel (II) steht R vorzugsweise für diejenigen Reste, die bereits im Zusammenhang mit der Beschreibung der erfindungsgemäßen Verbindungen der Formel (I) als bevorzugt für diesen Substituenten genannt wurden.

X steht in Formel (II) vorzugsweise für Fluor, Chlor oder Brom oder für geradkettiges oder verzweigtes Alkylsulfonyl mit 1 bis 4 Kohlenstoffatomen, insbesondere für Chlor oder Methylsulfonyl.

Die Benzoxazol-Derivate der Formel (II) sind bekannt oder erhältlich in Analogie zu bekannten Verfahren (vgl. z.B. EP 141 053; EP 43 573; DE 30 25 910; Am. Chem. J. 21, 111 [1899]; J. Prakt. Chem. (2) 42, 445 (1890); DE 11 64 413).

Die zur Durchführung des erfindungsgemäßen Verfahrens weiterhin als Ausgangsstoffe benötigten 2-Hydroxyacetamide sind durch die Formel (III) allgemein definiert. In dieser Formel (III) steht Ar vorzugsweise für diejenigen Reste, die bereits im Zusammenhang mit der Beschreibung der erfindungsgemäßen Verbindungen der Formel (I) als bevorzugt für diesen Substituenten genannt wurden.

Die 2-Hydroxyacetamide der Formel (III) sind ebenfalls bekannt (vgl. z.B. DE 38 21 600).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester; Sulfoxide, wie Dimethylsulfoxid oder Alkohole, wie Methanol, Ethanol, n- oder i-Propanol oder n-, i-, s- oder t-Butanol.

Das erfindungsgemäße Verfahren wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -40 °C und 120 °C, vorzugsweise bei Temperaturen zwischen -20 °C und 60 °C.

Zur Durchführung des erfindungsgemäßen Verfahrens setzt man pro Mol an Benzoxazol-Derivat der Formel (II) im allgemeinen 1,0 bis 2,0 Mol, vorzugsweise 1,0 bis 1,5 Mol an 2-Hydroxyacetamid der Formel (III) und gegebenenfalls 0,1 bis 2,0 Mol, vorzugsweise 1,0 bis 1,2 Mol an Reaktionshilfsmittel ein.
Die Reaktionsdurchführung, Aufarbeitung und Isolierung der Reaktionsprodukte erfolgt nach bekannten Verfahren (vgl. hierzu beispielsweise EP 348 734; EP 5501; DE 38 21 600 oder die Herstellungsbeispiele).

Die Reinigung der Endprodukte der Formel (I) erfolgt mit Hilfe üblicher Verfahren, beispielsweise durch Säulenchromatographie oder durch Umkristallisieren. Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von monokotylen Unkräutern in monokotylen und dikotylen Kulturen wie beispielsweise Baumwolle oder Reis einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionische und anionische Emulgatoren, wie Polyoxyethylen-Fettsäure-Ester, Polyoxyethylen-Fettalkohol-Ether, z.B. Alkylaryl-polygklykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es kommen in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil;

Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streu en.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1:

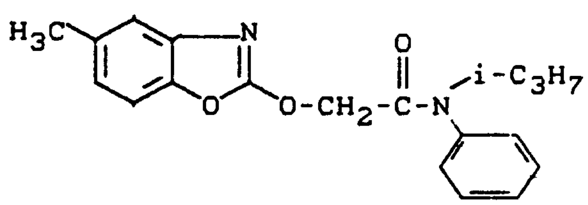

Zu 9,8 g (0,05 Mol) Hydroxyessigsäure-N-isopropyl-anilid und 7,6 g (0,045 Mol) 2-Chlor-5-methylbenzoxazol in 100 ml Aceton gibt man bei -20 °C tropfenweise unter Rühren eine Lösung aus 2,4 g (0,06 Mol) Natriumhydroxid in 10 ml Wasser und rührt anschließend 3 Stunden bei 0 bis 5 °C. Zur Aufarbeitung gibt man Wasser zu, rührt so lange bis das Produkt kristallin wird, saugt ab und trocknet.

Man erhält 13,6 g (85% der Theorie) 2-(5-Methyl-benzoxazol-2-yl-oxy)-N-isopropyl-acetanilid vom Schmelzpunkt 108 °C.

In entsprechender Weise und gemäß den allgemeinen Angaben zur Herstellung erhält man die folgenden 2-(2-Benzoxazolyl-oxy)-acetamide der allgemeinen Formel (I):

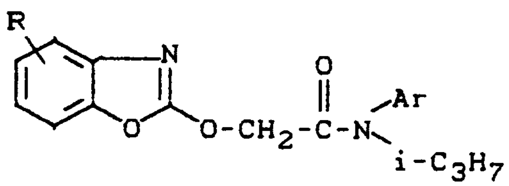

(I)

Tabelle 1

| Bsp.-Nr. | 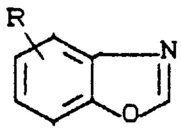 | Ar | Schmelzpunkt /°C |
|---|---|---|---|
| 2 | 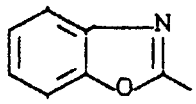 | 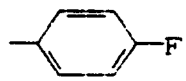 | 108 |
| 3 | 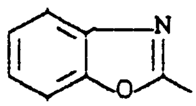 | 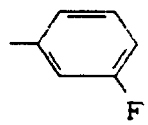 | 116 |
| 4 | 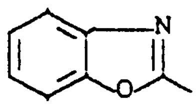 | 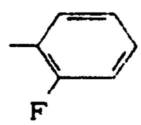 | 91 |
| 5 | 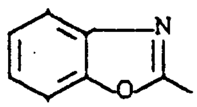 | 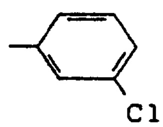 | 115 |
| 6 | 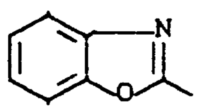 | 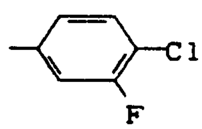 | 153 |

Tabelle 1

| Bsp.-Nr. | 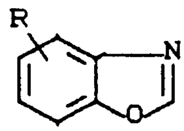 | Ar | Schmelzpunkt /°C |
|---|---|---|---|
| 7 | 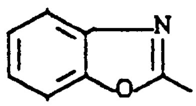 | 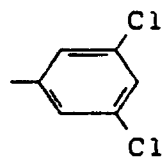 | 107 |
| 8 | 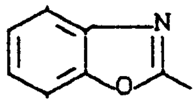 | 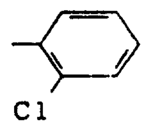 | 76 |
| 9 | 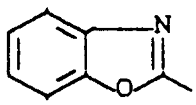 | 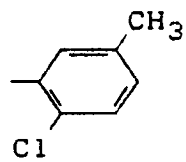 | 147 |
| 10 | 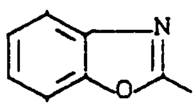 | 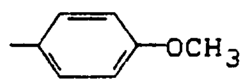 | 128 |
| 11 | 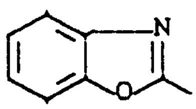 | 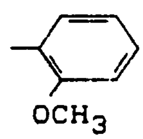 | 94 |
| 12 | 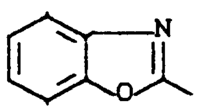 | 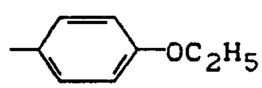 | 85 |
| 13 | 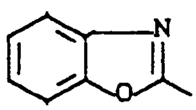 | 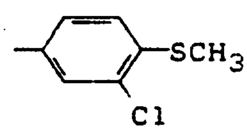 | 152 |

Tabelle 1

| Bsp.-Nr. | 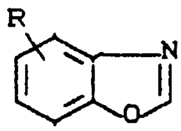 | Ar | Schmelzpunkt /°C |
|---|---|---|---|
| 14 | 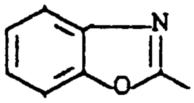 | 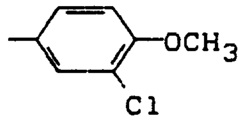 | 97 |
| 15 | 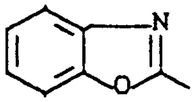 | 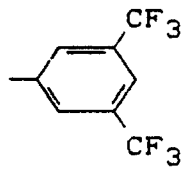 | 132 |
| 16 | 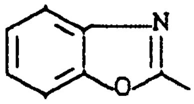 | 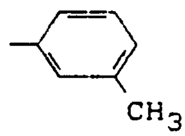 | 100 |
| 17 | 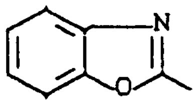 | 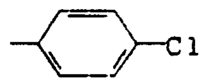 | 100 |
| 18 | 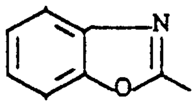 | 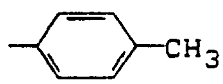 | 70 |
| 19 | 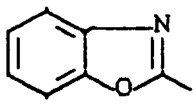 | 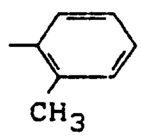 | 95 |
| 20 | 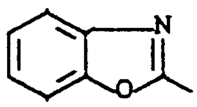 | 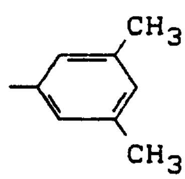 | 127 |

Tabelle 1

| Bsp.-Nr. | 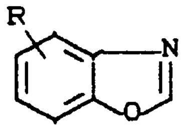 | Ar | Schmelzpunkt /°C |
|---|---|---|---|
| 21 | 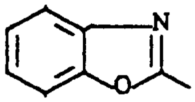 | 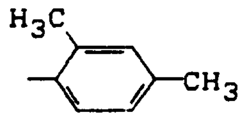 | 106 |
| 22 | 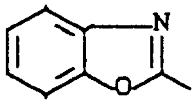 | 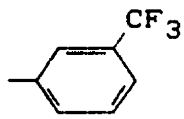 | 114 |
| 23 | 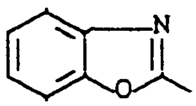 | 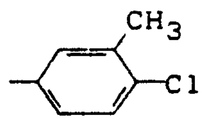 | 153 |
| 24 | 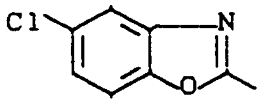 | 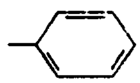 | 115 |
| 25 | 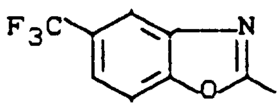 | 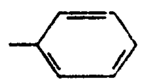 | 88 |
| 26 | 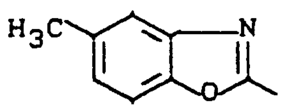 | 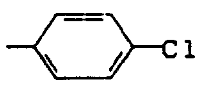 | 114 |
| 27 | 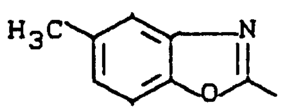 | 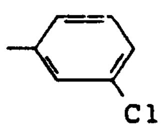 | 88 |
| 28 | 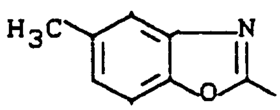 | 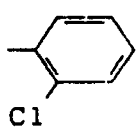 | 96 |

Tabelle 1

| Bsp.-Nr. | 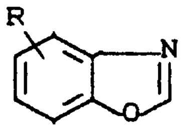 | Ar | Schmelzpunkt /°C |
|---|---|---|---|
| 29 | 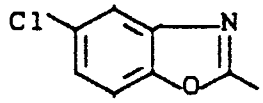 | 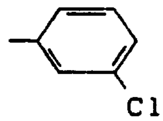 | 132 |
| 30 | 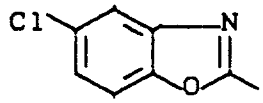 | 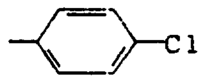 | 157 |
| 31 | 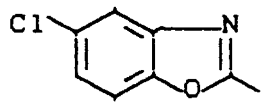 | 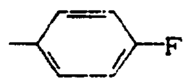 | 140 |
| 32 | 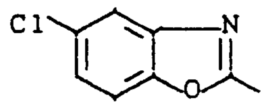 | 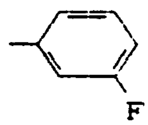 | 85 |
| 33 | 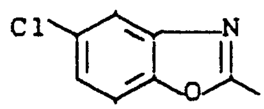 | 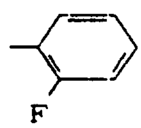 | 128 |
| 34 | 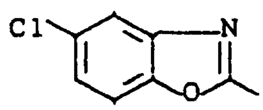 | 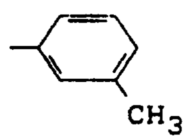 | 126 |
| 35 | 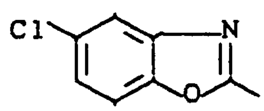 | 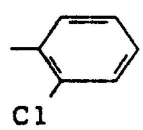 | 117 |

Tabelle 1

| Bsp.-Nr. | 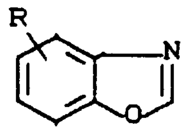 | Ar | Schmelzpunkt /°C |
|---|---|---|---|
| 36 | 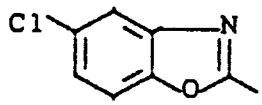 | 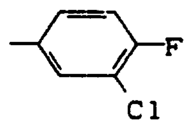 | 122 |
| 37 | 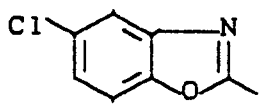 | 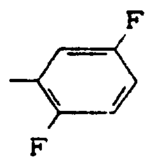 | 105 |
| 38 | 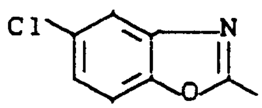 | 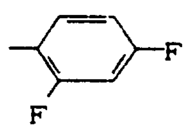 | 102 |
| 39 | 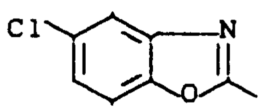 | 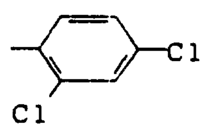 | 122 |
| 40 | 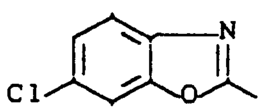 | 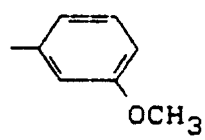 | 88 |
| 41 | 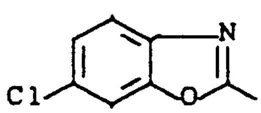 | 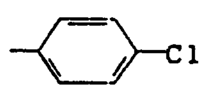 | 223 |

Tabelle 1

| Bsp.-Nr. | 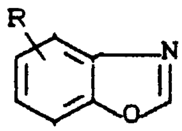 | Ar | Schmelzpunkt /°C |
|---|---|---|---|
| 42 | 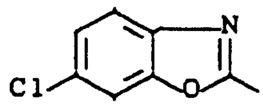 | 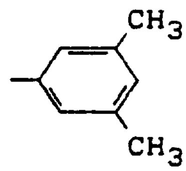 | 148 |
| 43 | 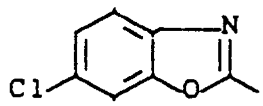 | 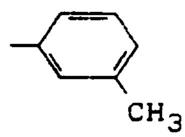 | 75 |
| 44 | 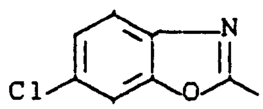 | 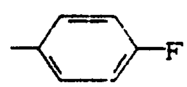 | 120 |
| 45 | 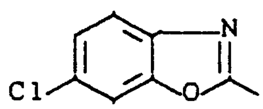 | 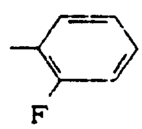 | 124 |
| 46 | 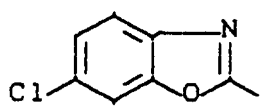 | 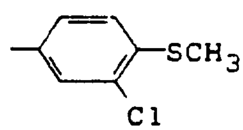 | 130 |
| 47 | 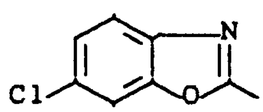 | 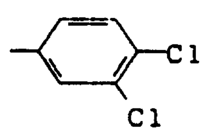 | 102 |
| 48 | 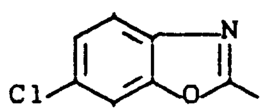 | 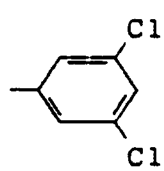 | 129 |

Tabelle 1

| Bsp.-Nr. | 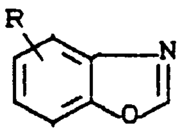 | Ar | Schmelzpunkt /° C |
|---|---|---|---|
| 49 | 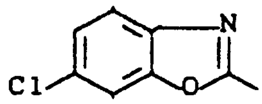 | 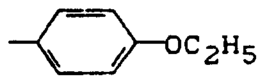 | 75 |
| 50 | 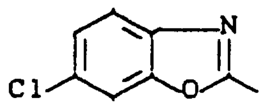 | 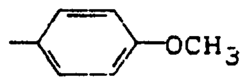 | 112 |
| 51 | 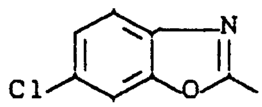 | 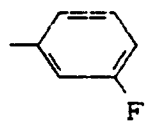 | 107 |
| 52 | 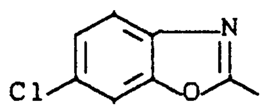 | 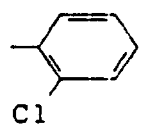 | 101 |
| 53 | 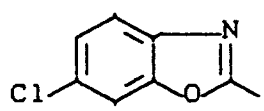 | 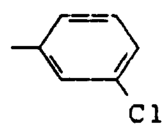 | 135 |
| 54 | 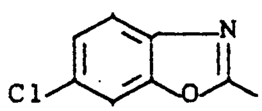 | 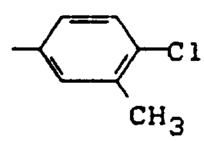 | 99 |
| 55 | 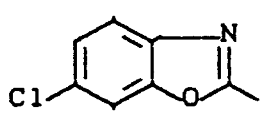 | 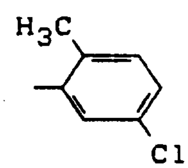 | 130 |

Tabelle 1

| Bsp.-Nr. | 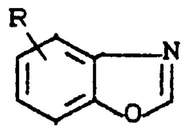 | Ar | Schmelzpunkt /° C |
|---|---|---|---|
| 56 | 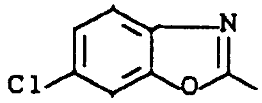 | 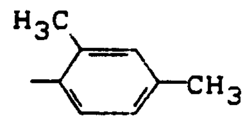 | 104 |
| 57 | 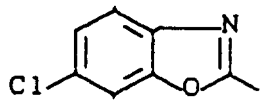 | 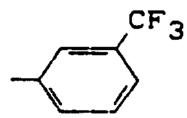 | 121 |
| 58 | 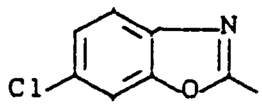 | 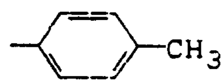 | 132 |
| 59 | 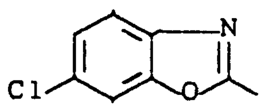 | 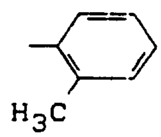 | 135 |
| 60 | 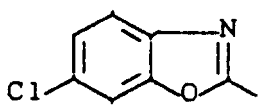 | 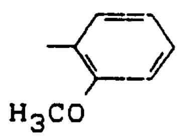 | 112 |
| 61 | 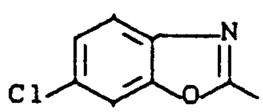 | 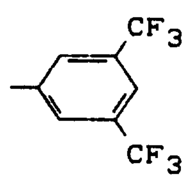 | 117 |

Anwendungsbeispiele:

In den folgenden Anwendungsbeispielen wurde die nachstehend aufgeführte Verbindung als Vergleichssubstanz eingesetzt:

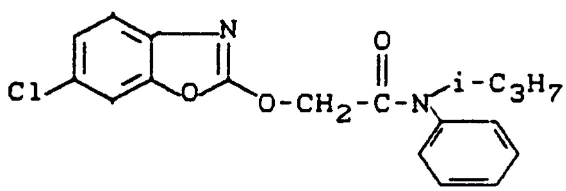 (A)

2-(6-Chlorbenzoxazol-2-yl-oxy)-N-isopropyl-acetanilid (vgl. z.B. DE 34 18 168; DE 37 24 467).

Beispiel A:

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber dem Stand der Technik zeigen in diesem Test z.B. die Verbindungen gemäß folgender Herstellungsbeispiele: 1, 2, 3, 5, 6, 7, 10, 12, 14, 16, 17, 18, 20, 22, 23, 26, 43, 44, 49, 51, 53, 54 und 58.

**Patentansprüche**

**1.** 2-(2-Benzoxyzolyl-oxy)-acetamide der allgemeinen Formel (I),

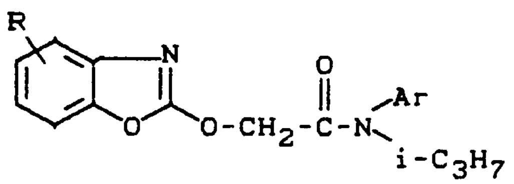

(I)

in welcher

R für Wasserstoff, Halogen, Alkyl oder Halogenalkyl steht und

Ar für gegebenenfalls substituiertes Aryl steht,

wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

**2.** 2-(2-Benzoxaolyl-oxy)-acetamide der allgemeinen Formel (I) gemäß Anspruch 1, dadurch gekennzeichnet, daß darin

R für Wasserstoff, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

Ar für gegebenenfalls einfach bis dreifach, gleich oder verschieden substituiertes Phenyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

**3.** 2-(2-Benzoxazolyl-oxy)-acetamide der allgemeinen Formel (I) gemäß Anspruch 1, dadurch gekennzeichnet, daß darin

R für Wasserstoff, Fluor, Chlor, Brom, für geradkettiges oder verzweigtes Alkyl mit 1 bis 6 Kohlenstoffatomen oder für geradkettiges oder verzweigtes Halogenalkyl mit 1 bis 3 Kohlenstoffatomen und 1 bis 7 gleichen oder verschiedenen Halogenatomen steht und

Ar für gegebenenfalls einfach bis dreifach, gleich oder verschieden substituiertes Phenyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

**4.** 2-(2-Benzoxazolyl-oxy)-acetamide der allgemeinen Formel (I) gemäß Anspruch 1, dadurch gekennzeichnet, daß darin

R für Wasserstoff, Chlor, Methyl, Ethyl, n- oder i-Propyl, n-, i-, s- oder t-Butyl oder Trifluormethyl steht und

Ar für gegebenenfalls einfach oder zweifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy und/oder Trifluormethylthio,

wobei die Verbindungen 2-(2-Benzoxazolyl-oxy)-N-isopropyl-acetanilid und 2-(6-Chlorbenzoxazol-2-yl-oxy)-N-isopropyl-acetanilid ausgenommen sind.

**5.** Verfahren zur Herstellung von 2-(2-Benzoxazolyloxy)-acetamiden der allgemeinen Formel (I) gemäß Anspruch 1, dadurch gekennzeichnet, daß man Benzoxazol-Derivate der Formel (II),

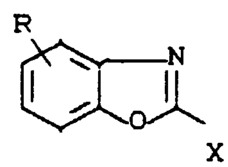

(II)

in welcher

X für Halogen oder Alkylsulfonyl steht und

R die in Anspruch 1 angegebene Bedeutung hat,

mit 2-Hydroxyacetamiden der Formel (III)

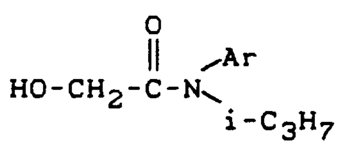

(III)

in welcher

Ar die in Anspruch 1 angegebene Bedeutung hat,

gegebenenfalls in Gegenwart eines Verdünnungsmittels und gegebenenfalls in Gegenwart eines Reaktionshilfsmittels umsetzt.

**6.** Herbizide Mittel, gekennzeichnet durch einen Gehalt an mindestens einer Verbindung der Formel (I) gemäß Anspruch 1.

**7.** Verwendung von Verbindungen der allgemeinen Formel (I) gemäß Anspruch 1 zur Bekämpfung von unerwünschtem Pflanzenwachstum.

**8.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**9.** Verfahren zur Herstellung von herbiziden Mitteln, dadurch gekennzeichnet, daß man Verbindungen der allgemeinen Formel (I) gemäß Anspruch 1 mit Streckmitteln und/oder oberflächlichen-aktiven Mitteln

vermischt.

Europäisches Patentamt

# EUROPÄISCHER RECHERCHENBERICHT

Nummer der Anmeldung

EP 92 11 6538

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | US-A-4 784 682 (HEINZ FÖRSTER ET AL)<br>* Ansprüche * | 1-9 | C07D263/58<br>A01N43/76 |
| X | EP-A-0 037 526 (NIHON TOKUSHU NOYAKU SEIZO K.K.)<br>* Seite 15, Zeile 15 - Seite 15, Zeile 26; Ansprüche * | 1-9 | |
| X | EP-A-0 062 254 (NIHON TOKUSHU NOYAKU SEIZO K.K.)<br>* Ansprüche * | 1-9 | |
| X | EP-A-0 037 938 (NIHON TOKUSHU NOYAKU SEIZO K.K.)<br>* Ansprüche * | 1-9 | |
| D,X | EP-A-0 005 501 (BAYER AKTIENGESELLSCHAFT)<br>* Ansprüche * | 1-9 | |
| D,X | DE-A-3 038 599 (BAYER AKTIENGESELLSCHAFT)<br>* Ansprüche * | 1-9 | RECHERCHIERTE SACHGEBIETE (Int. Cl.5) |
| D,X | DE-A-3 038 652 (BAYER AKTIENGESELLSCHAFT)<br>* Ansprüche * | 1-9 | C07D |

Der vorliegende Recherchenbericht wurde für alle Patentansprüche erstellt

| Recherchenort | Abschlußdatum der Recherche | Prüfer |
|---|---|---|
| DEN HAAG | 14 DEZEMBER 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)